# EUROPEAN PATENT SPECIFICATION

(11) **EP 4 680 094 B1**
(45) Date of publication and mention of the grant of the patent: **25.11.2026**
(21) Application number: 24710747.7
(22) Date of filing: 13.03.2024
(51) Int. Cl.: A61B 5/00, G01M 3/16, G03B 17/55, G03B 15/03, G03B 15/14

(54) **COOLING SYSTEM OF AN IMAGING HEAD OF A FLUORESCENCE IMAGING APPARATUS**
KÜHLSYSTEM EINES ABBILDUNGSKOPFES EINER FLUORESZENZABBILDUNGSVORRICHTUNG
SYSTÈME DE REFROIDISSEMENT D'UNE TÊTE D'IMAGERIE D'UN APPAREIL D'IMAGERIE PAR FLUORESCENCE

(30) Priority: 14.03.2023 EP 23161791
(43) Date of publication of application: 21.01.2026
(73) Proprietor: SurgVision GmbH, 81379 Munich (DE)
(72) Inventor: TARUTTIS, Adrian, 81379 Munich (DE); SHARAPOV, Anton, 81379 Munich (DE); DEN DEKKER, Harm, 5683 CR Best (NL); VELSINK, Florien, 5683 CR Best (NL); MARKS, Wouter, 5683 CR Best (NL)
(74) Representative: Pezzoli, Ennio
(86) International application number: PCT/EP2024/056699
(87) International publication number: WO 2024/189097

(56) References cited:
- KR-B1- 101 436 543
- US-A1- 2023 003 649
- US-A1- 2023 052 579
- US-B1- 8 622 896

## Description

### Technical field

The present disclosure relates to the field of medical equipment. More specifically, this disclosure relates to fluorescence imaging apparatus.

### Background art

The background of the present disclosure is hereinafter introduced with the discussion of techniques relating to its context. However, even when this discussion refers to documents, acts, artifacts and the like, it does not suggest or represent that the discussed techniques are part of the prior art or are common general knowledge in the field relevant to the present disclosure.

Imaging apparatus are commonly used in several medical applications to provide visual representations of body-parts of patients even if they are not visible directly. Particularly, imaging apparatus of fluorescence type exploit a fluorescence phenomenon occurring in fluorescence substances (called fluorophores), which emit fluorescence light when they are illuminated. The fluorescence light that is emitted from different locations of the body-parts may then be used to build fluorescence images representing the fluorophores that are present in the body-parts. For example, fluorescence agents (possibly adapted to reaching specific molecules of desired targets, such as lesions like tumors, and then to remaining immobilized thereon in Fluorescence Molecular Imaging (FMI) procedures) may be administered to the patients. The representation of the (immobilized) fluorescence agents in the corresponding fluorescence images facilitates the identification (and quantification) of the corresponding targets. This information may be used in several medical applications, for example, in surgical procedures for recognizing margins of lesions to be resected.

For this purpose, the imaging apparatus is provided with an imaging head for framing the body-parts to be imaged. The imaging head has an illumination unit that provides a (fluorescence) excitation light for exciting the fluorophores being present in the body-parts and an acquisition unit for acquiring the fluorescence images thereof.

The imaging head (and particularly its illumination unit) generates a significant amount of waste heat (as a byproduct of its operation). However, an excessive heating of the imaging head adversely affects its performance. Particularly, the excessive heating of the illumination unit causes a spread of characteristics (such as radiated power and wavelength) of the excitation light that generates corresponding noise in the fluorescence images. Therefore, a cooling system is generally provided to cool the imaging head so as to maintain a stable (relatively) low temperature thereof, and particularly of its illumination unit (ensuring a consistent illumination).

However, the cooling of the imaging head is quite challenging, especially in case of its use in surgical procedures.

Particularly, operating rooms have special ventilation requirements to prevent infections of surgical cavities being opened in the body-parts, often requiring laminar flow of air in a region thereof. Therefore, it is difficult to use forced air cooling systems wherein (cooler) air is forced to flow towards the imaging head for replacing (warmer) air to which the wasted heat has been transferred by convection; in fact, the position of the imaging head in close proximity to the body-parts hinders maintaining the laminar flow of air at their surgical cavities.

Moreover, contact of the surgical cavities has to be prevented by any non-sterile material. Therefore, infection risks for the patients may be caused by a liquid cooling system wherein the wasted heat is transferred to a (liquid) coolant that circulates through the imaging head; in fact, any leakage of the cooling system may cause the coolant (generally non-sterile) to fall onto the surgical cavities. More generally, the leakage of the coolant may pose safety risks for the patients depending on its toxicity.

In any case, the cooling system should have a size and a structure that interfere as little as possible with a handling of the imaging head for framing the body-parts to be imaged.

KR-A-101436543 discloses a fluorescence imaging system with a LED light source and a camera arranged at a through-hole thereof. A cooling device is provided at a rear end of the LED light source. A controller supplies cooling water to the cooling device through two circulation tubes connected thereto. However, this cooling device is quite ineffective in cooling the camera; moreover, it does not provide any protection against possible leakages of the cooling water.

Another cooling system for an imaging head according to the state of the art is for instance described in US8622896B1.

### Summary

The present invention is set out in the appended claims.

A simplified summary of the present disclosure is herein presented in order to provide a basic understanding thereof; however, the sole purpose of this summary is to introduce some concepts of the disclosure in a simplified form as a prelude to its following more detailed description, and it is not to be interpreted as an identification of its key elements nor as a delineation of its scope.

In general terms, the present disclosure is based on the idea of creating a chamber being sealed by a dissipator.

Particularly, an aspect provides an imaging head of a fluorescence imaging apparatus. The imaging head comprises a dissipator that is coupled with a container at an operative side thereof through a corresponding seal to define a chamber. An illumination unit is arranged on the dissipator outside the chamber. An acquisition unit is arranged inside the chamber; the acquisition unit is coupled with the dissipator at an opening thereof through a corresponding seal.

A further aspect provides a fluorescence imaging apparatus comprising the imaging head.

A further aspect provides a method for imaging a body-part of a patient with this imaging head.

A further aspect provides a corresponding medical method. The invention is defined in the independent claims and advantageous features thereof are set out in the dependent claims.

### Brief description of the drawings

The solution of the present disclosure, as well as further features and the advantages thereof, will be best understood with reference to the following detailed description thereof, given purely by way of a non-restrictive indication, to be read in conjunction with the accompanying drawings (wherein, for the sake of simplicity, corresponding elements are denoted with equal or similar references and their explanation is not repeated, and the name of each entity is generally used to denote both its type and its attributes, such as value, content and representation). In this respect, it is expressly intended that the drawings are not necessary drawn to scale (with some details that may be exaggerated and/or simplified) and that, unless otherwise indicated, they are merely used to illustrate the structures and procedures described herein conceptually. In addition, orientations and related position references (such as front, rear, upper, lower, lateral and so on) are to be understood in relation to a condition of use of the corresponding entities. Particularly:
FIG.1 shows a pictorial representation of a fluorescence imaging apparatus wherein the solution according to an embodiment of the present disclosure may be implemented,
FIG.2 shows a schematic representation in cross-section view of an imaging head of the fluorescence imaging apparatus according to an embodiment of the present disclosure, and
FIG.3-FIG.4 show an exploded view from different observation directions of the imaging head according to an embodiment of the present disclosure.

### Detailed description

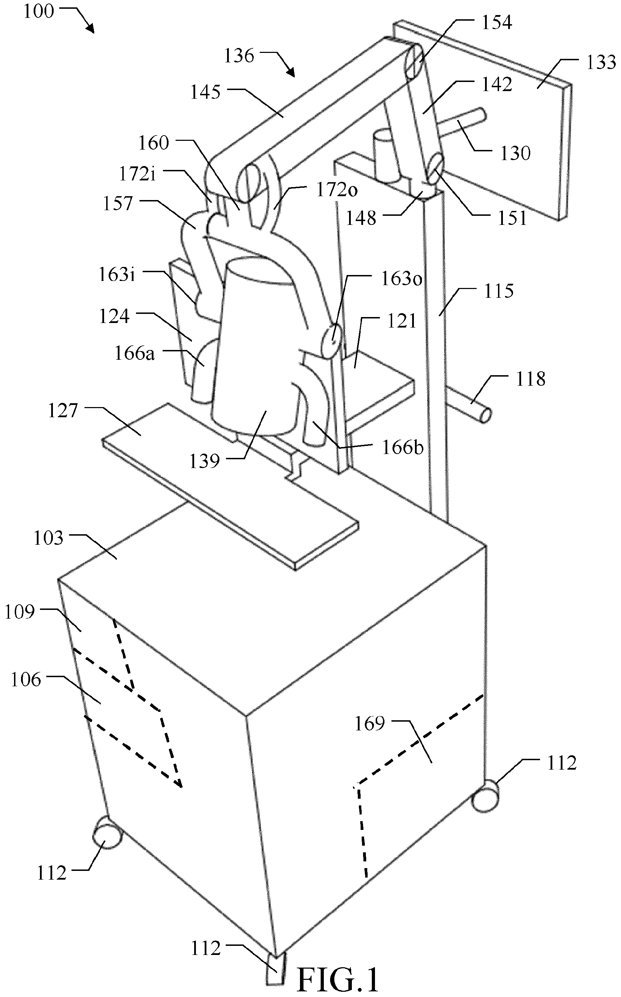

With reference in particular to FIG.1, a pictorial representation is shown of a fluorescence imaging apparatus 100 wherein the solution according to an embodiment of the present disclosure may be implemented.

The (fluorescence) imaging apparatus 100 is used in medical applications to inspect body-parts of patients (not shown in the figure) during imaging procedures, for example, in diagnostic, therapeutic and/or surgical procedures, by applying fluorescence imaging techniques. For example, the imaging apparatus 100 is used to assist a surgeon in Fluorescence Guided Surgery (FGS), and particularly Fluorescence Guided Resection (FGR) when relating to tumors.

The imaging apparatus 100 comprises the following components.

A cart 103 houses a (power) supply unit 106 and a control unit 109 for supplying and controlling, respectively, the imaging apparatus 100. Four casters 112 (only three visible in the figure) are arranged at corresponding lower corners of the cart 103 to facilitate moving the imaging apparatus 100 (with a foot brake, not shown in the figure, that is provided for securing the imaging apparatus 100 in position). A pillar 115 extends upwards from a back surface of the cart 103. The pillar 115 has a handlebar 118 for moving the imaging apparatus 100 by an operator thereof. A cantilever 121 projects from the pillar 115, above the cart 103. A primary monitor 124 (for displaying images to the operator) and a keyboard 127 with a pointing device such as a mouse or a trackball (for entering information/commands by the operator) are mounted on the cantilever 121. A pivoting arm 130 is mounted on top of the pillar 115 (above the cantilever 121). A secondary monitor 133 (for displaying images to a doctor, such as a surgeon) is mounted on the pivoting arm 130 (so as to allow turning it in any directions). An articulated arm 136 is mounted on top of the pillar 115 as well (next to the pivoting arm 130). An imaging head 139 (for framing the body-parts to be imaged) is suspended from the articulated arm 136.

For example, the articulated arm 136 comprises two links formed by corresponding bars 142 and 145. The bar 142 is coupled at an end thereof with the pillar 115 via two revolute joints 148 and 151 that allow it to rotate with respect thereto around a vertical axis and around a horizontal axis, respectively. The bar 145 is coupled at an end thereof with another end of the bar 142 via a revolute joint 154 that allows it to rotate with respect thereto around a horizontal axis. A support of the imaging head 139 formed by a fork 157 is coupled at a common point of its prongs with another end of the bar 145 via a revolute joint 160 that allows it to rotate with respect thereto around a vertical axis. The imaging head 139 is coupled at its center with the prongs of the fork 157 via corresponding revolute joints 163i and 163o that allow it to rotate with respect thereto around a horizontal axis (representing a pitch axis of the imaging head 139). In this way, the imaging head 139 has 5 degrees of freedom, since it may translate in space (forward-backward, leftward-rightward and upward-downward) via the (revolute) joints 148-154, it may rotate around the vertical axis via the (revolute) joint 160 and it may rotate around its pitch axis via the (revolute) joints 163i,163o. The imaging head 139 is provided with two handlebars 166a and 166b for positioning it by the operator.

A heat exchanger, for example, a chiller 169 is further housed within the cart 103. An inlet duct 172i and an outlet duct 172o (only partially visible in the figure) circulate a coolant fluid, or simply coolant, between the chiller 169 and the imaging head 139. The coolant is a substance with a relatively high thermal capacity, which accumulates and conveys heat; preferably, the coolant is in the liquid state (for example, water). Particularly, the inlet duct 172i supplies, *i.e.,* pumps (cooler) coolant to the imaging head 139 for cooling it (as described in detail in the following), thereby warming up. The outlet duct 172o returns the (warmer) coolant to the chiller 169 that removes heat therefrom (dispersing it into the external environment). For this purpose, the (inlet/outlet) ducts 172i,172o are thermally insulated (to limit heat dissipation) and flexible (to follow the movements of the imaging head 139); for example, the ducts 172i,172o run between the chiller 169 and the pillar 115 inside the cart 130, run inside the pillar 115, pass between the pillar 115 and the articulated arm 136 outside the joints 148-151, run inside the bar 142, pass between the bar 142 and the bar 145 outside the joint 154, run inside the bar 145, pass between the bar 145 and the fork 157 outside the joint 160 and pass between the fork 157 and the imaging head 139 through the joints 163i,163o. In this way, the chiller 169 may be maintained relatively far away from the patients during the imaging procedures with a beneficial effect on their safety; for example, in surgical procedures this allows avoiding (or at least substantially reducing) the chiller 169 from adversely affecting special ventilation requirements of a corresponding surgery room (being required to prevent infections of surgical cavities being opened in the body-parts), such as maintaining laminar flow of air in a region thereof.

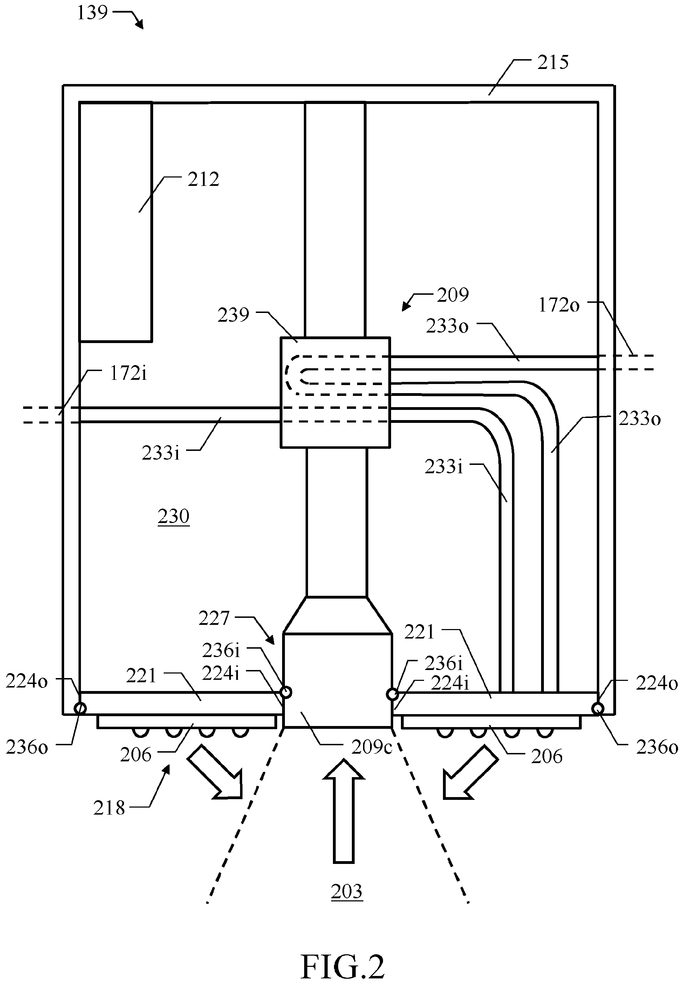

With reference now to FIG.2, a schematic representation is shown in cross-section view of the imaging head 139 (of the fluorescence imaging apparatus) according to an embodiment of the present disclosure.

The imaging head 139 is configured for imaging a scene comprised in a field of view 203 thereof (defined by a part of the world within a solid angle to which the imaging head 139 is sensitive). Particularly, in case of a surgical procedure the scene (not shown in the figure) relates to a patient to whom a fluorescence agent has been previously administered (for example, comprising fluorophores adapted to accumulating in a corresponding target, such as tumors); the scene comprises a body-part of the patient, wherein a surgical cavity (for example, a small skin incision in minimally invasive surgery) has been opened to expose a lesion (such as a tumor) to be resected.

The imaging head 139 comprises the following components.

An illumination unit 206, an acquisition unit 209 and service components 212 define a functional assembly of the imaging head 139 (implementing its function). Particularly, the illumination unit 206 is used to illuminate the scene of the field of view 203. The illumination unit 206 generates a (fluorescence) excitation light and possibly a white light; the excitation light has wavelength and energy suitable to excite the fluorophores of the fluorescence agent (such as of Near Infra-Red (NIR) type), whereas the white light appears substantially colorless to the human eye (such as containing all the wavelengths of the spectrum that is visible to the human eye at equal intensity). The acquisition unit 209 is used to acquire (digital) images of the scene of the field of view 203. The acquisition unit 209 acquires fluorescence images that are defined by fluorescence light being emitted by the fluorophores of the fluorescence agent when they are illuminated by the excitation light (then representing the corresponding target within the body-part). In fact, the fluorophores pass to an excited (electronic) state when they absorb the excitation light; the excited state is unstable, so that the fluorophores very shortly decay therefrom to a ground (electronic) state, thereby emitting the fluorescence light with an intensity mainly depending on the amount of the fluorophores that are illuminated. Moreover, the acquisition unit 209 may also acquire reflectance (or photograph) images that are defined by a visible light being reflected by a content of the field of view 203 illuminated by the white light (then representing what is visible to the human eye in the field of view 203). Particularly, the acquisition unit 209 comprises collection optics 209c that is used to collect light from the field of view 203, and other components used to generate the fluorescence/reflectance images from the collected light (for example, a dichroic mirror splitting the collected light into the fluorescence light and the visible light, filters to remove any residual components of the fluorescence/visible lights, a fluorescence camera generating the fluorescence images from the fluorescence light, a reflectance camera generating the reflectance images from the visible light and so on). The collection optics 209c is arranged at a center of the illumination unit 206 (so as to ensure optimal operation of the imaging head 139). The service components 212 comprise one or more (active/passive) components supporting operation of the illumination unit 206 and of the acquisition unit 209 (for example, electronic components such as a voltage regulator, power switches and the like, fuses and so on). A container 215 (for example, of plastic material) has an operative side 218 for exposing the illumination unit 206 and the acquisition unit 209. In the specific embodiment shown in the figure, the container 215 is in the form of a bell (such as with generically cylindrical shape) with its operative side 218 defined by a (lower) mouth (*i.e.,* a cavity for accessing the container 215). The acquisition unit 209 and the service components 212 are arranged in the container 215. The illumination unit 206 is arranged at the operative side 218 of the container 215 (the mouth of the bell in this case), with the collection optics 209c passing through it. The imaging head 139 has a cooling system for cooling it, and particularly its illumination unit 206, acquisition unit 209 and service components 212.

A dissipator 221, in the form of a dissipation plate, is provided. The dissipator 221 is made of a thermally conductive material (such as aluminum) for transferring heat (as described in the following), with an annulus-like shape matching the container 215 and the collection optics 209c (circular in the example at issue). Particularly, the dissipator 221 has a size corresponding to the container 215 at its operative side 218 (the mouth of the bell in this case), with an outer edge 224o matching it. A (through) hole 227 corresponding to the collection optics 209c (with a circular shape in the example at issue) defines an opening at a center of the dissipator 221, with an inner edge 224i matching it. The dissipator 221 is coupled with the container 215 at its operative side 218 (the mouth of the bell in this case) so as to define a chamber 230. The illumination unit 206 is arranged on the dissipator 221, outside the chamber 230 (below it in the figure). The acquisition unit 209 and the service components 212 are arranged inside the chamber 230, with the collection optics 209c operating through the hole 227. An inlet duct 233i and an outlet duct 233o are further arranged inside the chamber 230 for circulating the coolant for the dissipator 221. Particularly, the inlet duct 233i runs from the inlet duct 172i to the dissipator 221 to supply (cooler) coolant thereto from the chiller (not shown in the figure) for cooling it, thereby warming up; the outlet duct 233o runs from the dissipator 221 to the outlet duct 172o to return the (warmed) coolant to the chiller that removes heat therefrom. An outer seal 236o acts between the outer edge 224o of the dissipator 221 and the container 215 at its operative side 218 (the mouth of the bell in this case); likewise, an inner seal 236i acts between the inner edge 224i of the dissipator 221 (at its hole 227) and the collection optics 209c. The (inner/outer) seals 236o,236i help the coupling of the dissipator 221 with the container 215 and the collection optics 209c to prevent escape of the coolant from the container 215 in case of a leakage of the cooling system in correspondence to the (inlet/outlet) ducts 233i,233o, for example, along the ducts 233i,233o and/or at their connections to the ducts 172i,172o and to the dissipator 221. During the imaging procedures, the illumination unit 206 and the collection optics 209c (and then the dissipator 221 as well) generally faces downwards, either vertically or tilted. Therefore, in this way at least a lower part (in operative conditions) of the imaging head 139 is sealed so as to hold the coolant inside the chamber 230 even in case of a leakage of the cooling system.

The above-described structure is very effective in cooling the imaging head 139. Particularly, the heat generated by the illumination unit 206 is transferred by conduction to the dissipator 221, wherein it is absorbed by the coolant circulating through it. In addition, the coolant circulating in the chamber 230 (through the dissipator 221 and the ducts 233i,233o) cools the air encapsulated therein by convection and radiation, which (cooled) air then absorbs the heat generated by the acquisition unit 209 and the service components 212 as well.

All of the above allows maintaining a stable (relatively) low temperature of the whole imaging head 139 with a beneficial effect on its operation (particularly, of the illumination unit 206 providing a consistent illumination of the field of view 203, of the acquisition unit 209 providing a reduced noise of the fluorescence images, and of the service components 212 providing a correct working thereof).

The sealing of the chamber 230 ensures that the coolant cannot escape from it even in case of a leakage of the cooling system (ducts 233i,233o and/or corresponding connections), at least in a normal orientation of the imaging head 139 during the imaging procedures (generally, with the illumination unit 206 and the collection optics 209c facing downwards). This prevents the coolant from falling onto the body-parts being imaged, thereby avoiding any risk for a health of the patients (even if the coolant was toxic), and especially infection risks in surgical procedures wherein the coolant (being typically non-sterile) might enter the surgical cavities.

The desired result is achieved in a relatively simple way. In fact, the dissipator 221 fits with the structure of the imaging head 139 (and particularly with the arrangement of the illumination unit 206 and the collection optics 209c); moreover, the sealing of the chamber 230 is obtained by exploiting the container 215 that is already available in the imaging head 139.

As a further improvement, the imaging head 139 comprises one or more (additional) dissipators 239. Particularly, in the specific embodiment of the figure only a single dissipator 239 is shown being coupled with the acquisition unit 209 (for example, in the form of a bush embracing it). The dissipator 239 is again made of a thermally conductive material (such as aluminum) for transferring heat. The dissipator 239 is coupled with the inlet duct 233i and the outlet duct 233o for circulating the coolant through it as well, with the inlet duct 233i running from the inlet duct 172i to the dissipator 239 to supply (cooler) coolant thereto from the chiller for cooling it and the outlet duct 233o running from the dissipator 239 to the outlet duct 172o to return the (warmed) coolant to the chiller (either directly or indirectly). Particularly, in the example shown in the figure the inlet duct 233i runs from the inlet duct 172i to the dissipator 239 and then from the dissipator 239 to the dissipator 221, whereas the outlet duct 233o runs from the dissipator 221 to the dissipator 239 and then from the dissipator 239 to the outlet duct 172o.

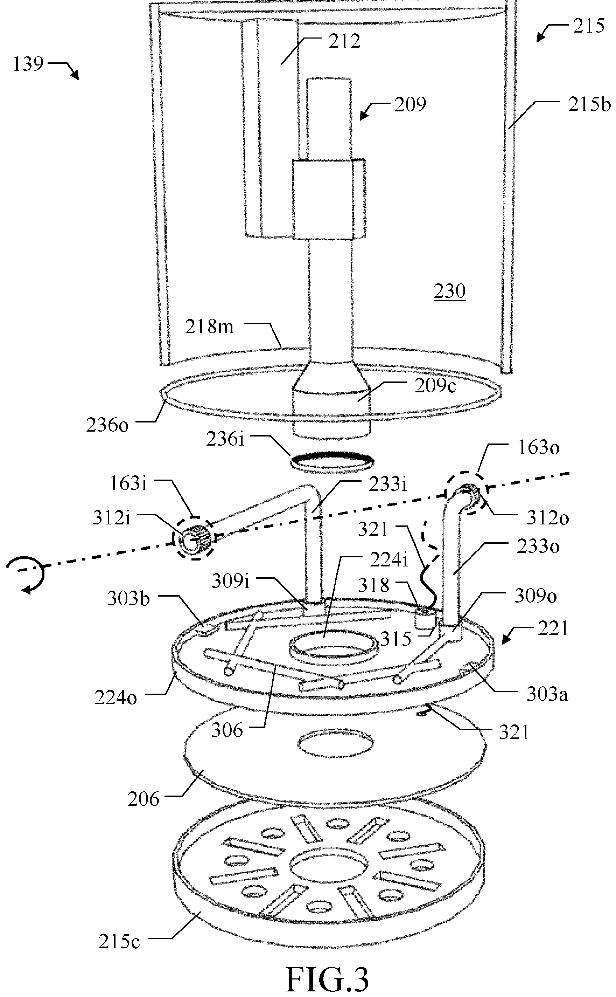

With reference now to FIG.3-FIG.4, an exploded view is shown from different observation directions of the imaging head 139 according to an embodiment of the present disclosure.

Starting from FIG.3 (showing the imaging head 139 in its normal orientation during the imaging procedures, with the illumination unit 206 and the collection optics 209c facing downwards), the outer seal 236o and the inner seal 236i are implemented by corresponding gaskets (for example, O-rings), which are seated into matching grooves at the outer edge 224o and at the inner edge 224i, respectively, of the dissipator 221. The (outer/inner) gaskets 236o,236i are made of a deformable material (for example, an elastomer); when the imaging head 139 is assembled, the outer gasket 236o and the inner gasket 236i are compressed between the dissipator 221 and the container 215 (shown in partially cut-away view in the figure) and between the dissipator 221 and the collection optics 209c, respectively, so as to seal their interfaces. This structure is quite simple but at the same time effective; moreover, it allows opening/closing the imaging head 139 in a relatively simple way (for example, for maintenance operations).

One or more sensors 303a,303b are provided inside the chamber 230. The sensors 303a,303b are connected electrically to the control unit of the imaging apparatus via corresponding cables (not shown in the figure), which run from the sensors 303a,303b to the control unit through the joints 163i,163, the articulated arm, the pillar and the cart of the imaging apparatus (not shown in the figure). The sensors 303a,303b detect any presence of a liquid, and particularly of the coolant, in the chamber 230. For this purpose, each sensor 303a,303b generates a presence signal in case the coolant reaches it (because of a leakage of the cooling system). The control unit receives any presence signal from the sensors 303a,303b (for example, by polling them). In response thereto, the control unit enters an alarm condition. Particularly, the control unit stops the supply of the coolant to the imaging head 139 by the chiller (not shown in the figure), for example, via a corresponding relay; this avoids providing further coolant to the imaging head 139, thereby limiting its leakage. In addition, the control unit switches off a power supply of the imaging head 139, for example, via a corresponding power switch; this avoids possible short-circuits that might be caused by the coolant. At the same time, the control unit warns the operator accordingly, for example, by outputting a corresponding message on one or more of the monitors of the imaging apparatus (not shown in the figure). This significantly reduces the risks of damages to the imaging head 139 in case of a leakage of the cooling system; moreover, it allows the operator to intervene promptly to secure the environment (for example, by removing the imaging head 139 from a corresponding patient).

Preferably, the sensors 303a,303b are multiple and arranged at different positions in the chamber 230 for detecting the presence of the coolant in corresponding orientations of the imaging head 139. Particularly, in the example at issue the imaging head may translate (with 3 degrees of freedom), may rotate around the vertical axis and may rotate around its (horizontal) pitch axis passing through the joints 163i and 163o (as shown in the figure by a corresponding arrow); moreover, the imaging head 139 is normally used with the illumination unit 206 and the collection optics 209c facing downwards. Therefore, in this case two sensors 303a and 303b are enough to detect the leakage of the coolant in any orientation of the imaging head 139. Particularly, the sensors 303a,303b are positioned on the dissipator 221 at opposite ends thereof, and extend along a (sensing) direction that is perpendicular to the pitch axis. In fact, in case of a leakage the coolant deposits onto the dissipator 221 by gravity. When the imaging head 139 extends vertically, both sensors 303a and 303b detect the coolant; when the imaging head is rotated clockwise (in the same direction of the arrow) the coolant accumulates on the right of the dissipator 221 in the figure so that it is detected by the sensor 303a, whereas when the imaging head is rotated counterclockwise (in the opposite direction of the arrow) the coolant accumulates on the left of the dissipator 221 in the figure so that it is detected by the sensor 303b. This allows detecting any leakage of the coolant promptly at its earlier stage (irrespectively of the orientation of the imaging head 139).

The dissipator 221 is provided with a circulation circuit 306 for the coolant (for example, formed by a series of communicating conduits that are connected in series, four straight tubes in the example shown in the figure). The circulation circuit 306 runs inside the chamber 230 on an inner surface of the dissipator 221, for example, being mounted thereon (such as screwed), from an inlet connector 309i (to which the inlet duct 233i is connected) to an outlet connector 309o (to which the outlet duct 233o is connected). This provides a good cooling of the dissipator 221, at the same time facilitating the cooling of the air in the chamber 230 (and then of the acquisition unit 209 and of the service components 212).

The ducts 233i,233o are made of a material with a high thermal conductivity (for example, aluminum), and extend along at least part of the imaging head 139. For example, the ducts 233i and 233o are connected between the connectors 309i and 309o, respectively, at the dissipator 221 (lower end of the imaging head 139) and further connectors 312i and 312o, respectively, at the joints 163i and 163o, respectively (center of the imaging head 139). This further facilitates the cooling of the air in the chamber 230, and then of the acquisition unit 209 and of the service components 212.

The dissipator 221 has a further, smaller, (through) hole 315 defining a corresponding opening, with a grommet 318 for passing an (electrical) cable 321 (or more). The grommet 318 is made of a deformable material (for example, an elastomer); the grommet 318 is press-fitted into the hole 315 (so as to seal an interface between them) and is provided with a (through) hole crossing the hole 315, which has a width smaller than a section of the cable 321. Therefore, the cable 321 is inserted under pressure through the grommet 318, so that the grommet 318 is compressed thereby sealing an interface between them. The cable 321 electrically connects the illumination unit 206 to the control unit (for supplying/controlling it); for this purpose, the cable 321 runs from the control unit through the cart, the pillar, the articulated arm, the joints 163i,163o and the grommet 318. This allows connecting the illumination unit 206 electrically inside the imaging head 139, at the same time maintaining its (at least partial) sealing, preventing the coolant from escaping from the imaging head 139 even in case of a leakage of the cooling system.

The container 215 of the imaging head 139 also comprises, in addition to the bell, denoted with the reference 215b, a cap 215c that is mounted on the bell 215b at its mouth, denoted with the reference 218m. The cap 215c protects the illumination unit 206, at the same time exposing its functional components (as described in the following).

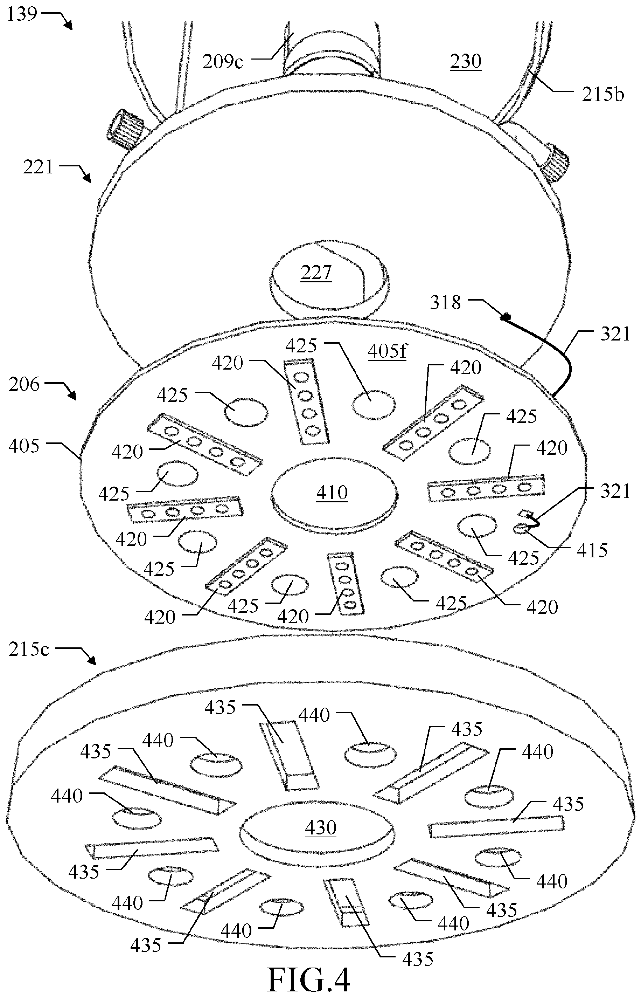

Moving to FIG.4 (showing the imaging head 139 in bottom view), the illumination unit 206 comprises a Printed Circuit Board (PCB), or simply board 405 (formed by a substrate of electrically insulating material with one or more layers of tracks of electrically conductive material). The board 405 matches the dissipator 221 (with an annulus-like shape as well in the example at issue); particularly, the board 405 has a (through) hole 410 corresponding to the hole 227 that defines an opening at a center thereof. A (through) hole 415 defining an opening for the cable 321 is formed in the board 405, at the same radial position of the grommet 318 in the dissipator 221. The board 405 is fastened outside the chamber 230 (only partially visible in the figure) onto an outer surface of the dissipator 221 (for example, mounted thereon, such as screwed), co-axially thereto (with the hole 410 around the hole 227) and rotated to have the hole 415 substantially co-axial with the grommet 318. A plurality of (excitation) light sources 420 providing the excitation light (for example, based on LEDs) are mounted on a front surface 405f of the board 405 (opposite the dissipator 221); for example, the lights sources 420 are arranged uniformly in a plurality of alignments extending radially (such as 8 alignments each of 4 light sources in the example shown in the figure) that are mounted on corresponding tracks of the board 405. A plurality of (white) light sources 425 providing the white light (for example, based on LEDs as well) may also be mounted on the front surface 405f of the board 405; for example, the light sources 425 are interposed among the alignments of the light sources 420. The cable 321 coming from the grommet 318 of the dissipator 221 runs to the front surface 405f of the board 405 through the hole 415. The cable 321 comprises a plurality of conductors (not shown in the figure) that are connected to corresponding tracks of the board 405 (in turn connected to the light sources 420,425).

The cap 215c is provided with a window 430 that defines an opening matching the hole 227 (at a center thereof), a plurality of windows 435 that define corresponding openings matching the light sources 420 and a plurality of windows 440 that define corresponding openings matching the light sources 425 (around the window 430). The cap 215c is mounted on the bell 215b (only partially visible in the figure) so that the window 430 is coaxial with the hole 227 (and the hole 410), rotated to have the windows 435 and 440 substantially co-axial with the light sources 420 and 425, respectively. In this way, the cap 215c covers the board 405 (and particularly its tracks), so as to protect it. At the same time, the windows 435 and 440 expose the light sources 420 and 425, respectively, so as to allow them to illuminate the field of view of the imaging head 139; moreover, the window 430 exposes the collection optics 209c (through the hole 227 and the hole 410), so as to allow it to acquire the fluorescence images and the reflectance images of the field of view of the imaging head 139.

In operation, the imaging head 139 may also be enclosed in a sterile drape (not shown in the figure). The sterile drape is a single-use (disposable) cover made of flexible and transparent material that has been sterilized; for example, a (new) sterile drape is used during each surgical procedure to further reduce the risks of infection of the corresponding patient. At the same time, the sterile drape may be used to wrap the imaging head 139 so as to provide an additional protection against any leakage of the coolant.

### Modifications

In order to satisfy local and specific requirements, a person skilled in the art may apply many logical and/or physical modifications and alterations to the present disclosure, while the invention is defined by the scope of the claims. More specifically, although this disclosure has been described with a certain degree of particularity with reference to one or more embodiments thereof, it should be understood that various omissions, substitutions and changes in the form and details as well as other embodiments are possible. Particularly, different embodiments of the present disclosure may be practiced even without the specific details (such as the numerical values) set forth in the preceding description to provide a more thorough understanding thereof; conversely, well-known features may have been omitted or simplified in order not to obscure the description with unnecessary particulars. Moreover, it is expressly intended that specific elements and/or method steps described in connection with any embodiment of the present disclosure may be incorporated in any other embodiment as a matter of general design choice. Moreover, items presented in a same group and different embodiments, examples or alternatives are not to be construed as *de facto* equivalent to each other (but they are separate and autonomous entities). In any case, each numerical value should be read as modified according to applicable tolerances; particularly, unless otherwise indicated, the terms "substantially", "about", "approximately" and the like should be understood as within 10%, preferably 5% and still more preferably 1%. Moreover, each range of numerical values should be intended as expressly specifying any possible number along the *continuum* within the range (comprising its end points). Ordinal or other qualifiers are merely used as labels to distinguish elements with the same name but do not by themselves connote any priority, precedence or order. The terms include, comprise, have, contain, involve and the like should be intended with an open, non-exhaustive meaning (*i.e.,* not limited to the recited items); the terms based on, dependent on, according to, function of and the like should be intended as a non-exclusive relationship (*i.e.,* with possible further variables involved); the term a/an should be intended as one or more items (unless expressly indicated otherwise); and the term means for (or any means-plus-function formulation) should be intended as any structure adapted or configured for carrying out the relevant function.

For example, an embodiment provides an imaging head of a fluorescence imaging apparatus. However, the imaging head may be of any fluorescence imaging apparatus (see below).

In an embodiment, the imaging head is for imaging a body-part of a patient in a medical imaging application. However, the imaging head may be used for imaging any body-part of any patient in any medical imaging application (see below).

In an embodiment, the imaging head is used for imaging the body-part of the patient when suspended over the patient. However, the imaging head may be configured to be suspended over the patient in any way (for example, mounted on any suspension structure, hand-held, end-effector of a robotic system or co-manipulator, and so on).

In an embodiment, the imaging head comprises an illumination unit for illuminating a field of view of the imaging head with a fluorescence excitation light. However, the illumination unit may be of any type (for example, with any number and type of light sources, such as LEDs, OLEDs, LECs, lasers and the like, arranged in any way, such as radially, circumferentially and the like, with or without additional white light sources of any type, and so on) and for providing any fluorescence excitation light (for example, NIR, Infra-Red (IR), visible and so on).

In an embodiment, the imaging head comprises an acquisition unit for acquiring fluorescence images of the field of view. However, the acquisition unit may be of any type (for example, based on any collection optics, EMCCD, CMOS, InGaAs, PMT and the like sensors, with or without a reflectance camera, and so on) and for acquiring any number and type of fluorescence images (for example, having any size/resolution, with any frame-rate, stand-alone or overlaid on corresponding reflectance images, and so on).

In an embodiment, the imaging head comprises a container having an operative side for exposing the illumination unit and the acquisition unit. However, the container may be of any type (for example, of any material, shape, size and structure, such as a bell closed by a cap, a vessel closed by a top, two half-shells joined together and so on).

In an embodiment, the imaging head comprises a dissipator for dissipating heat. However, the dissipator may be of any type (for example, of any material, with any thickness, with or without fins and so on).

In an embodiment, the dissipator comprises a plate having an opening, an outer edge and an inner edge at the opening. However, the opening may be of any type (for example, with any size, shape, at any position and so on) and the outer/inner edges may be of any type (for example, with corresponding grooves, flat and so on).

In an embodiment, the dissipator is coupled with the container at the operative side thereof to define a chamber. However, the dissipator may be coupled with the container in any way (for example, snap-fitted, screwed, at any position corresponding to the operative side, such as at its border or slightly inside, and so on) to define any chamber (for example, with any shape, size, completely sealed or only in part, and so on).

In an embodiment, the illumination unit is arranged on the dissipator outside the chamber. However, the illumination unit may be arranged on the dissipator in any way (for example, mounted, glued, soldered and the like, covering its whole extent or only part thereof, surrounding its opening completely or only partially, and so on).

In an embodiment, the acquisition unit is arranged inside the chamber to operate through the opening. However, the acquisition unit may be arranged inside the chamber in any way (for example, extending along its entire length or only in part, further extending or not laterally, and so on) and it may operate through the opening in any way (for example, flush with, protruding from or sunken into the dissipator, and so on).

In an embodiment, the imaging head comprises an inlet duct and an outlet duct for circulating a coolant fluid of the dissipator. However, the inlet/outlet ducts may be of any type (for example, with any size, cross-section, flexible/rigid, running completely outside or at least in part within the dissipator along any path, and so on) for circulating any coolant fluid (for example, a liquid, such as water, a mixture of water and glycol and the like, a gas and so on).

In an embodiment, the inlet duct and the outlet duct are arranged inside the chamber. However, the inlet/outlet ducts may be arranged inside the chamber in any way (for example, extending in any direction, for any extent and so on).

In an embodiment, the imaging head comprises an outer seal acting between the outer edge of the dissipator and the container and an inner seal acting between the inner edge of the dissipator and the acquisition unit for preventing the coolant fluid from falling onto the patient in case of a leakage thereof. However, the outer seal and the inner seal may be of any type (for example, for making the corresponding interfaces liquid-proof or gas-proof, each implemented by a gasket, such as an O-ring, a sheet gasket, a spiral-wound gasket and the like, an adhesive, a bonding and so on).

Further embodiments provide additional advantageous features, which may however be omitted at all in a basic implementation. In this respect, it is expressly intended that the features of each of the following embodiments may be combined with the above features either alone or in combination with the features of any number of the other following embodiments.

In an embodiment, the container comprises a bell having a mouth at the operative side. However, the bell may be of any type (for example, cylindrical, flared and so on) and it may have any mouth (for example, circular, squared and the like, at any position so on).

In an embodiment, the container comprises a cap being mounted on the bell at the mouth thereof. However, the cap may be of any type (for example, flat, rounded and so on) and it may be mounted on the bell in any way (for example, screwed, snap-fitted and so on).

In an embodiment, the cap has an opening exposing the acquisition unit. However, the opening of the cap may be of any type (for example, either the same or different with respect to the opening of the dissipator).

In an embodiment, the cap has one or more further openings exposing the illumination unit. However, the further openings of the cap may be in any number and of any type (for example, with any size and shape, either the same or different among them, at any position and so on).

In an embodiment, the imaging head comprises one or more sensors arranged inside the chamber for detecting a leakage of the coolant fluid. However, the sensors may be in any number, of any type (for example, printed sensors, sensor cables, flow meters and so on) and at any position (for example, at the dissipator, throughout the chamber, along the inlet/outlet ducts and so on).

In an embodiment, the sensors are arranged at the dissipator. However, this result may be achieved in any way (for example, with the sensors mounted on the dissipator, on the container close thereto and so on).

In an embodiment, the sensors are a plurality of sensors that are arranged at different positions for detecting the leakage of the coolant fluid in corresponding orientations of the imaging head. However, the sensors may be in any number and arranged at any positions for any corresponding orientations of the imaging head (for example, rotations around any number of axes being horizontal, inclined and so on).

In an embodiment, the sensors comprise two sensors arranged at opposite ends of the dissipator along a sensing direction perpendicular to a longitudinal axis of the imaging head. However, the sensing direction may be of any type (for example, extending laterally, transversally and so on).

In an embodiment, the imaging head comprises a circulation circuit of the coolant fluid being coupled with the inlet duct and the outlet duct. However, the circulation circuit may be of any type (for example, running along any path, such as a broken line, a curve and the like, formed by a single conformed element or by any number of interconnected elements, and so on).

In an embodiment, the circulation circuit is provided on the dissipator inside the chamber. However, the circulation circuit may be provided on the dissipator in any way (for example, mounted, glued, soldered and so on).

In an embodiment, the inlet duct and the outlet duct are configured to extend along at least part of the imaging head for cooling air inside the chamber. However, the inlet/outlet ducts may extend along the chamber in any way (for example, along its entire length or only in part, with any path and so on).

In an embodiment, the imaging head comprises one or more further dissipators for dissipating heat. However, the further dissipators may be in any number and of any type (for example, of any material, structure, size and so on).

In an embodiment, the further dissipators are coupled with the inlet duct and the outlet duct for circulating the coolant fluid. However, the dissipator and the further dissipators may be coupled with the inlet/outlet ducts in any way (for example, in series and/or in parallel, connecting each dissipator with corresponding connectors at the container directly or through one or more other dissipators, in any order among them, and so on).

In an embodiment, at least one of the further dissipators is arranged to dissipate heat produced by the acquisition unit. However, the dissipators acting on the acquisition unit may be in any number with any number of other dissipators acting on different components of the imaging head (for example, the sensors, the service components and so on), down to none in each case; moreover, each dissipator acting on the acquisition unit may be of any type (for example, arranged around, at a side and so on).

In an embodiment, the dissipator comprises a further opening. However, the further opening may be of any type (for example, with any size, shape, at any position and so on).

In an embodiment, the further opening is provided with a grommet. However, the grommet may be of any type (for example, with any shape, size, of any material and so on).

In an embodiment, the grommet is for sealing a passage of at least one electrical cable connected to the illumination unit. However, the grommet is for any number and type of electrical cables (for example, for providing a power supply, sending control signals, receiving response signals and so on); in any case, the possibility is not excluded of electrically contacting the illumination unit outside the chamber.

An embodiment provides a fluorescence imaging apparatus for imaging a body-part of a patient in a medical imaging application comprising the imaging head of above. However, the fluorescence imaging apparatus may be of any type (for example, a guided surgery equipment, a scanner and so on) for use in any medical imaging applications (for example, surgery, diagnostics, therapy and so on).

In an embodiment, the fluorescence imaging apparatus comprises a suspension structure for suspending the imaging head over the patient. However, the suspension structure may be of any type (for example, any articulated arm, such as mounted on a cart, a ceiling, a wall and the like, a cantilever, a hook and so on).

In an embodiment, the fluorescence imaging apparatus comprises a heat exchanger for cooling the coolant fluid. However, the heat exchanger may be of any type (for example, a chiller, a heat pump, with fluids in co-current, counter-current or cross-current, and so on).

In an embodiment, the heat exchanger is separated from the imaging head. However, the heat exchanger may be separated from the imaging head in any way (for example, arranged in the cart, remote from it and so on).

In an embodiment, the fluorescence imaging apparatus comprises a further inlet duct (for supplying the coolant fluid from the heat exchanger to the inlet duct) and a further outlet duct (for returning the coolant fluid from the outlet duct to the heat exchanger). However, the further inlet/outlet ducts may be of any type (for example, with any size, cross-section, flexible/rigid, standing alone or running at least in part inside one or more components of the imaging apparatus, such as its articulated arm, pillar, cart and so on).

In an embodiment, the fluorescence imaging apparatus comprises a control unit. However, the control unit may be of any type (for example, a microcontroller, a personal computer and so on).

In an embodiment, the control unit is coupled with the sensors. However, the control unit may be coupled with the sensors in any way (for example, with a wired connection running in any way, a wireless connection and so on).

In an embodiment, the control unit is coupled with the sensors for receiving an indication of said sensing the leakage of the coolant fluid. However, the control unit may receive this information in any way (for example, by polling the sensors, listening for notifications therefrom and so on).

In an embodiment, the control unit is configured for entering an alarm condition in response to said sensing the leakage of the coolant fluid. However, the control unit may enter the alarm condition in any way (for example, as soon as a sensor detects the coolant, after this is confirmed two or more times, in case any sensor does not respond, and so on) for performing any number and type of actions (for example, stopping the supply of the coolant fluid, switching off the power supply of the imaging head, outputting any visual and/or acoustic warnings, and so on).

In an embodiment, the control unit is configured to stop said supplying the coolant fluid from the heat exchanger to the inlet duct in the alarm condition. However, the supply of the coolant fluid may be stopped in any way (for example, switching off the heat exchanger, closing the further inlet duct and so on).

In an embodiment, the control unit is configured to switch off a power supply of the imaging head in the alarm condition. However, the power supply may be switched off in any way (for example, automatically, requiring a manual confirmation and so on).

In an embodiment, the suspension structure comprises one or more joints for rotating the imaging head around a lateral axis perpendicular to the longitudinal axis thereof (the sensors extending along the sensing direction being perpendicular to the lateral axis). However, this result may be achieved in any way (for example, with one or more rotational joints, a spherical joint and so on) for rotating the imaging head around any lateral axis (for example, a pitch axis, a yaw axis, a roll axis and so on).

Generally, similar considerations apply if the imaging head and the fluorescence imaging apparatus each has a different structure or comprises equivalent components, or it has other operative characteristics, provided that it remains within the scope of the claims. In any case, every component thereof may be separated into more elements, or two or more components may be combined together into a single element; moreover, each component may be replicated to support the execution of the corresponding operations in parallel. Moreover, unless specified otherwise, any interaction between different components generally does not need to be continuous, and it may be either direct or indirect through one or more intermediaries.

An embodiment provides a method for imaging a body-part of a patient in a medical imaging application. However, the method may be used for imaging any body-part (for example, one or more organs, a region thereof or tissues, in any pathological/health condition and so on) and of any patient (for example, a human being, an animal and so on) in any medical imaging application (see above). In any case, although the method may facilitate the task of a doctor, it only provides intermediate results that may help him/her but with the medical activity *stricto sensu* that is always made by the doctor himself/herself.

In an embodiment, the method comprises acquiring one or more fluorescence images of the body-part with the imaging head of above. However, the fluorescence images may be in any number; moreover, the same considerations pointed out above with respect to the features of the imaging head apply to the corresponding steps of the method as well.

In an embodiment, the method comprises outputting a representation of the body-part based on the fluorescence images. However, the representation of the body-part may be of any type (for example, the fluorescence images, the fluorescence images combined with any reflectance images and so on) and it may be outputted in any way (for example, displayed on any device, such as a monitor, virtual reality glasses and the like, or more generally output in real-time or off-line in any way, such as printed, transmitted remotely and so on).

## Claims

1. An imaging head (139) of a fluorescence imaging apparatus (100) for imaging a body-part of a patient in a medical imaging application when suspended over the patient, wherein the imaging head (139) comprises:
an illumination unit (206) for illuminating a field of view (203) of the imaging head (139) with a fluorescence excitation light,
an acquisition unit (209) for acquiring fluorescence images of the field of view (203),
a container (215,215c) having an operative side (218) for exposing the illumination unit (206) and the acquisition unit (209),
a dissipation plate (221) for dissipating heat having an opening (227), an outer edge (224o) and an inner edge (224i) at the opening (227), wherein the dissipation plate (221) is coupled with the container (215) at the operative side (218) thereof to define a chamber (230), the illumination unit (206) being arranged on the dissipation plate (221) outside the chamber (230) and the acquisition unit (209) being arranged inside the chamber (230) to operate through the opening (227),
an inlet duct (233i) and an outlet duct (233o) for circulating a coolant fluid through a circulation circuit (306) of the dissipation plate (221), the inlet duct (233i) and the outlet duct (233o) being arranged inside the chamber (230),
an outer seal (236o) acting between the outer edge (224o) of the dissipation plate (221) and the container (215,215c) and an inner seal (236i) acting between the inner edge (224i) of the dissipation plate (221) and the acquisition unit (209) for preventing the coolant fluid from falling onto the patient in case of a leakage thereof in correspondence to the inlet duct (233i) and/or the outlet duct (233o).

2. The imaging head (139) according to claim 1, wherein the container (215) comprises a bell (215b) having a mouth (218m) at the operative side and a cap (215c) being mounted on the bell (215b) at the mouth (218m) thereof, the cap (215c) having an opening (430) exposing the acquisition unit (209) and one or more further openings (435,440) exposing the illumination unit (206).

3. The imaging head (139) according to claim 1 or 2, wherein the imaging head (139) comprises one or more sensors (303a,303b) arranged inside the chamber (230) for detecting a leakage of the coolant fluid.

4. The imaging head (139) according to claim 3, wherein the sensors (303a,303b) are arranged at the dissipator (221).

5. The imaging head (139) according to claim 3 or 4, wherein the sensors (303a,303b) are a plurality of sensors (303a,303b) arranged at different positions for detecting the leakage of the coolant fluid in corresponding orientations of the imaging head (139).

6. The imaging head (139) according to claim 5, wherein the sensors (303a,303b) comprise two sensors (303a,303b) arranged at opposite ends of the dissipation plate (221) along a sensing direction perpendicular to a longitudinal axis of the imaging head (139).

7. The imaging head (139) according to any claim from 1 to 6, wherein the circulation circuit (306) is provided on the dissipation plate (221) inside the chamber (230).

8. The imaging head (139) according to any claim from 1 to 7, wherein the inlet duct (233i) and the outlet duct (233o) are configured to extend along at least part of the imaging head (139) for cooling air inside the chamber (230).

9. The imaging head (139) according to any claim from 1 to 8, wherein the imaging head (139) comprises one or more dissipators (239) for dissipating heat being coupled with the inlet duct (233i) and the outlet duct (233o) for circulating the coolant fluid, at least one of the dissipators (239) being arranged to dissipate heat produced by the acquisition unit (209).

10. A fluorescence imaging apparatus (100) for imaging a body-part of a patient in a medical imaging application, wherein the fluorescence imaging apparatus (100) comprises the imaging head (139) according to any claim from 1 to 9 and a suspension structure (136) for suspending the imaging head (100) over the patient.

11. The fluorescence imaging apparatus (100) according to claim 10, wherein the fluorescence imaging apparatus (100) comprises a heat exchanger (169) for cooling the coolant fluid being separated from the imaging head (139), a further inlet duct (172i) for supplying the coolant fluid from the heat exchanger (169) to the inlet duct (233i) and a further outlet duct (172o) for returning the coolant fluid from the outlet duct (233o) to the heat exchanger (169).

12. The fluorescence imaging apparatus (100) according to claim 10 or 11 when comprising the imaging head (139) according to any claim from 3 to 9, wherein the fluorescence imaging apparatus (100) comprises a control unit (109) coupled with the sensors (303a,303b) for receiving an indication of said sensing the leakage of the coolant fluid, the control unit (109) being configured for entering an alarm condition in response to said sensing the leakage of the coolant fluid.

13. The fluorescence imaging apparatus (100) according to claim 12 when dependent on claim 11, wherein the control unit (109) is configured to stop said supplying the coolant fluid from the heat exchanger (169) to the inlet duct (233i) and/or to switch off a power supply of the imaging head (139) in the alarm condition.

14. The fluorescence imaging apparatus (100) according to claim 12 or 13 when comprising the imaging head (139) according to any claim from 5 to 9, wherein the suspension structure (136) comprises one or more joints (163i,163o) for rotating the imaging head (139) around a lateral axis perpendicular to the longitudinal axis thereof, the sensors (303a,303b) extending along the sensing direction being perpendicular to the lateral axis.

15. A method for imaging a body-part of a patient in a medical imaging application, wherein the method comprises:
acquiring one or more fluorescence images of the body-part with the imaging head according to any claim from 1 to 9, and
outputting a representation of the body-part based on the fluorescence images.

## Patentansprüche

1. Bildgebungskopf (139) einer Fluoreszenzbildgebungsvorrichtung (100) zur Bildgebung eines Körperteils eines Patienten in einer medizinischen Bildgebungsanwendung, wenn er über dem Patienten aufgehängt ist, wobei der Bildgebungskopf (139) Folgendes umfasst:
eine Beleuchtungseinheit (206) zum Beleuchten eines Sichtfelds (203) des Bildgebungskopfs (139) mit einem Fluoreszenzanregungslicht,
eine Erfassungseinheit (209) zum Erfassen von Fluoreszenzbildern des Sichtfelds (203),
einen Behälter (215, 215c) mit einer operativen Seite (218) zum Freilegen der Beleuchtungseinheit (206) und der Erfassungseinheit (209),
eine Ableitplatte (221) zum Ableiten von Wärme mit einer Öffnung (227), einer Außenkante (224o) und einer Innenkante (224i) an der Öffnung (227), wobei die Ableitplatte (221) mit dem Behälter (215) an dessen operativen Seite (218) gekoppelt ist, um eine Kammer (230) zu definieren, wobei die Beleuchtungseinheit (206) auf der Ableitplatte (221) außerhalb der Kammer (230) angeordnet ist und die Erfassungseinheit (209) innerhalb der Kammer (230) angeordnet ist, um durch die Öffnung (227) zu arbeiten,
einen Einlasskanal (233i) und einen Auslasskanal (233o) zum Zirkulieren eines Kühlfluids durch einen Zirkulationskreislauf (306) der Ableitplatte (221), wobei der Einlasskanal (233i) und der Auslasskanal (233o) innerhalb der Kammer (230) angeordnet sind,
eine Außendichtung (236o), die zwischen der Außenkante (224o) der Ableitplatte (221) und dem Behälter (215, 215c) wirkt, und eine Innendichtung (236i), die zwischen der Innenkante (224i) der Ableitplatte (221) und der Erfassungseinheit (209) in Übereinstimmung mit dem Einlasskanal (233i) und/oder dem Auslasskanal (233o) wirkt, um zu verhindern, dass das Kühlfluid im Falle einer Leckage desselben auf den Patienten fällt.

2. Bildgebungskopf (139) nach Anspruch 1, wobei der Behälter (215) eine Glocke (215b) mit einer Mündung (218m) an der operativen Seite und eine Haube (215c), die an der Glocke (215b) an deren Mündung (218m) montiert ist, umfasst, wobei die Haube (215c) eine Öffnung (430), die die Erfassungseinheit (209) freilegt, und eine oder mehrere weitere Öffnungen (435, 440), die die Beleuchtungseinheit (206) freilegen, aufweist.

3. Bildgebungskopf (139) nach Anspruch 1 oder 2, wobei der Bildgebungskopf (139) einen oder mehrere Sensoren (303a, 303b) umfasst, die innerhalb der Kammer (230) angeordnet sind, um eine Leckage des Kühlfluids zu detektieren.

4. Bildgebungskopf (139) nach Anspruch 3, wobei die Sensoren (303a, 303b) an dem Ableiter (221) angeordnet sind.

5. Bildgebungskopf (139) nach Anspruch 3 oder 4, wobei es sich bei den Sensoren (303a, 303b) um eine Vielzahl von Sensoren (303a, 303b) handelt, die an verschiedenen Positionen angeordnet sind, um die Leckage des Kühlfluids in entsprechenden Ausrichtungen des Bildgebungskopfs (139) zu detektieren.

6. Bildgebungskopf (139) nach Anspruch 5, wobei die Sensoren (303a, 303b) zwei Sensoren (303a, 303b) umfassen, die an gegenüberliegenden Enden der Ableitplatte (221) entlang einer Erkennungsrichtung senkrecht zu einer Längsachse des Bildgebungskopfs (139) angeordnet sind.

7. Bildgebungskopf (139) nach einem der Ansprüche 1 bis 6, wobei der Zirkulationskreislauf (306) an der Ableitplatte (221) innerhalb der Kammer (230) bereitgestellt ist.

8. Bildgebungskopf (139) nach einem der Ansprüche 1 bis 7, wobei der Einlasskanal (233i) und der Auslasskanal (233o) dazu konfiguriert sind, sich entlang mindestens eines Teils des Bildgebungskopfs (139) zum Kühlen von Luft innerhalb der Kammer (230) zu erstrecken.

9. Bildgebungskopf (139) nach einem der Ansprüche 1 bis 8, wobei der Bildgebungskopf (139) einen oder mehrere Ableiter (239) zum Ableiten von Wärme umfasst, die mit dem Einlasskanal (233i) und dem Auslasskanal (233o) gekoppelt sind, um das Kühlfluid zu zirkulieren, wobei mindestens einer der Ableiter (239) so angeordnet ist, dass er von der Erfassungseinheit (209) erzeugte Wärme ableitet.

10. Fluoreszenzbildgebungsvorrichtung (100) zur Bildgebung eines Körperteils eines Patienten in einer medizinischen Bildgebungsanwendung, wobei die Fluoreszenzbildgebungsvorrichtung (100) den Bildgebungskopf (139) nach einem der Ansprüche 1 bis 9 und eine Aufhängungsstruktur (136) zum Aufhängen des Bildgebungskopfs (100) über dem Patienten umfasst.

11. Fluoreszenzbildgebungsvorrichtung (100) nach Anspruch 10, wobei die Fluoreszenzbildgebungsvorrichtung (100) einen Wärmetauscher (169) zum Kühlen des Kühlfluids, der von dem Bildgebungskopf (139) getrennt ist, einen weiteren Einlasskanal (172i) zum Zuführen des Kühlfluids von dem Wärmetauscher (169) zu dem Einlasskanal (233i) und einen weiteren Auslasskanal (172o) zum Rückführen des Kühlfluids von dem Auslasskanal (233o) zu dem Wärmetauscher (169) umfasst.

12. Fluoreszenzbildgebungsvorrichtung (100) nach Anspruch 10 oder 11, wenn sie den Bildgebungskopf (139) nach einem der Ansprüche 3 bis 9 umfasst, wobei die Fluoreszenzbildgebungsvorrichtung (100) eine Steuereinheit (109) umfasst, die mit den Sensoren (303a, 303b) gekoppelt ist, um eine Anzeige des Erkennens der Leckage des Kühlfluids zu empfangen, wobei die Steuereinheit (109) dazu konfiguriert ist, als Reaktion auf das Erkennen der Leckage des Kühlfluids in einen Alarmzustand einzutreten.

13. Fluoreszenzbildgebungsvorrichtung (100) nach Anspruch 12, wenn abhängig von Anspruch 11, wobei die Steuereinheit (109) dazu konfiguriert ist, das Zuführen des Kühlfluids von dem Wärmetauscher (169) zu dem Einlasskanal (233i) zu stoppen und/oder eine Stromversorgung des Bildgebungskopfs (139) in dem Alarmzustand abzuschalten.

14. Fluoreszenzbildgebungsvorrichtung (100) nach Anspruch 12 oder 13, wenn sie den Bildgebungskopf (139) nach einem der Ansprüche 5 bis 9 umfasst, wobei die Aufhängungsstruktur (136) ein oder mehrere Gelenke (163i, 163o) umfasst, um den Bildgebungskopf (139) um eine Querachse senkrecht zu seiner Längsachse zu drehen, wobei sich die Sensoren (303a, 303b) entlang der Erkennungsrichtung erstrecken, die senkrecht zu der Querachse ist.

15. Verfahren zur Bildgebung eines Körperteils eines Patienten in einer medizinischen Bildgebungsanwendung, wobei das Verfahren Folgendes umfasst:
Erfassen eines oder mehrerer Fluoreszenzbilder des Körperteils mit dem Bildgebungskopf nach einem der Ansprüche 1 bis 9 und
Ausgeben einer Darstellung des Körperteils basierend auf den Fluoreszenzbildern.

## Revendications

1. Tête d'imagerie (139) d'un appareil d'imagerie par fluorescence (100) permettant de prendre en image une partie du corps d'un patient dans une application d'imagerie médicale lorsqu'elle est suspendue au-dessus du patient, dans laquelle la tête d'imagerie (139) comprend :
une unité d'éclairage (206) destinée à éclairer un champ de vision (203) de la tête d'imagerie (139) avec une lumière d'excitation de fluorescence,
une unité d'acquisition (209) destinée à acquérir des images de fluorescence du champ de vision (203),
un conteneur (215,215c) présentant un côté opérationnel (218) destiné à exposer l'unité d'éclairage (206) et l'unité d'acquisition (209),
une plaque de dissipation (221) destinée à dissiper la chaleur, présentant une ouverture (227), un bord extérieur (224o) et un bord intérieur (224i) au niveau de l'ouverture (227), dans laquelle la plaque de dissipation (221) est couplée au conteneur (215) au niveau du côté opérationnel (218) de celui-ci pour définir une chambre (230), l'unité d'éclairage (206) étant agencée sur la plaque de dissipation (221) à l'extérieur de la chambre (230) et l'unité d'acquisition (209) étant agencée à l'intérieur de la chambre (230) pour fonctionner à travers l'ouverture (227),
un conduit d'entrée (233i) et un conduit de sortie (233o) destinés à faire circuler un fluide de refroidissement à travers un circuit de circulation (306) de la plaque de dissipation (221), le conduit d'entrée (233i) et le conduit de sortie (233o) étant agencés à l'intérieur du chambre (230),
un joint extérieur (236o) agissant entre le bord extérieur (224o) de la plaque de dissipation (221) et le conteneur (215,215c) et un joint intérieur (236i) agissant entre le bord intérieur (224i) de la plaque de dissipation (221) et l'unité d'acquisition (209) pour empêcher le fluide de refroidissement de tomber sur le patient en cas de fuite de celui-ci au niveau du conduit d'entrée (233i) et/ou du conduit de sortie (233o).

2. Tête d'imagerie (139) selon la revendication 1, dans laquelle le conteneur (215) comprend une cloche (215b) présentant une embouchure (218m) au niveau du côté opérationnel et un capuchon (215c) monté sur la cloche (215b) au niveau de l'embouchure (218m) de celle-ci, le capuchon (215c) présentant une ouverture (430) exposant l'unité d'acquisition (209) et une ou plusieurs ouvertures supplémentaires (435,440) exposant l'unité d'éclairage (206).

3. Tête d'imagerie (139) selon la revendication 1 ou 2, dans laquelle la tête d'imagerie (139) comprend un ou plusieurs capteurs (303a,303b) agencés à l'intérieur de la chambre (230) pour détecter une fuite du fluide de refroidissement.

4. Tête d'imagerie (139) selon la revendication 3, dans laquelle les capteurs (303a, 303b) sont agencés au niveau du dissipateur (221).

5. Tête d'imagerie (139) selon la revendication 3 ou 4, dans laquelle les capteurs (303a,303b) sont une pluralité de capteurs (303a,303b) agencés à différentes positions pour détecter la fuite du fluide de refroidissement dans des orientations correspondantes de la tête d'imagerie (139).

6. Tête d'imagerie (139) selon la revendication 5, dans laquelle les capteurs (303a, 303b) comprennent deux capteurs (303a,303b) agencés au niveau d'extrémités opposées de la plaque de dissipation (221) le long d'une direction de détection perpendiculaire à un axe longitudinal de la tête d'imagerie (139).

7. Tête d'imagerie (139) selon l'une quelconque des revendications 1 à 6, dans laquelle le circuit de circulation (306) est prévu sur la plaque de dissipation (221) à l'intérieur de la chambre (230).

8. Tête d'imagerie (139) selon l'une quelconque des revendications 1 à 7, dans laquelle le conduit d'entrée (233i) et le conduit de sortie (233o) sont conçus pour s'étendre le long d'au moins une partie de la tête d'imagerie (139) afin de refroidir l'air à l'intérieur de la chambre (230).

9. Tête d'imagerie (139) selon l'une quelconque des revendications 1 à 8, dans laquelle la tête d'imagerie (139) comprend un ou plusieurs dissipateurs (239) destinés à dissiper la chaleur, couplés au conduit d'entrée (233i) et au conduit de sortie (233o) pour faire circuler le fluide de refroidissement, au moins l'un des dissipateurs (239) étant agencé pour dissiper la chaleur produite par l'unité d'acquisition (209).

10. Appareil d'imagerie par fluorescence (100) destiné à prendre en image une partie du corps d'un patient dans une application d'imagerie médicale, dans lequel l'appareil d'imagerie par fluorescence (100) comprend la tête d'imagerie (139) selon l'une quelconque des revendications 1 à 9 et une structure de suspension (136) destinée à suspendre la tête d'imagerie (100) au-dessus du patient.

11. Appareil d'imagerie par fluorescence (100) selon la revendication 10, dans lequel l'appareil d'imagerie par fluorescence (100) comprend un échangeur de chaleur (169) destiné à refroidir le fluide de refroidissement qui est séparé de la tête d'imagerie (139), un conduit d'entrée supplémentaire (172i) destiné à acheminer le fluide de refroidissement de l'échangeur de chaleur (169) au conduit d'entrée (233i) et un conduit de sortie supplémentaire (172o) destiné à renvoyer le fluide de refroidissement du conduit de sortie (233o) à l'échangeur de chaleur (169).

12. Appareil d'imagerie par fluorescence (100) selon la revendication 10 ou 11, lorsqu'il comprend la tête d'imagerie (139) selon l'une quelconque des revendications 3 à 9, dans lequel l'appareil d'imagerie par fluorescence (100) comprend une unité de commande (109) couplée aux capteurs (303a,303b) pour recevoir une indication de ladite détection de la fuite du fluide de refroidissement, l'unité de commande (109) étant configurée pour entrer dans un état d'alarme en réponse à ladite détection de la fuite du fluide de refroidissement.

13. Appareil d'imagerie par fluorescence (100) selon la revendication 12 lorsqu'elle dépend de la revendication 11, dans lequel l'unité de commande (109) est configurée pour arrêter ladite alimentation en fluide de refroidissement de l'échangeur de chaleur (169) vers le conduit d'entrée (233i) et/ou pour couper une alimentation électrique de la tête d'imagerie (139) dans l'état d'alarme.

14. Appareil d'imagerie par fluorescence (100) selon la revendication 12 ou 13, lorsqu'il comprend la tête d'imagerie (139) selon l'une quelconque des revendications 5 à 9, dans lequel la structure de suspension (136) comprend un ou plusieurs joints (163i,163o) destinés à faire tourner la tête d'imagerie (139) autour d'un axe latéral perpendiculaire à l'axe longitudinal de celle-ci, les capteurs (303a,303b) s'étendant le long de la direction de détection étant perpendiculaires à l'axe latéral.

15. Procédé permettant de prendre en image une partie du corps d'un patient dans une application d'imagerie médicale, dans lequel le procédé comprend :
l'acquisition d'une ou plusieurs images de fluorescence de la partie du corps au moyen de la tête d'imagerie selon l'une quelconque des revendications 1 à 9, et
la livraison en sortie d'une représentation de la partie du corps sur la base des images de fluorescence.
